(19) Europäisches Patentamt
European Patent Office
Office européen des brevets

(11) Publication number: 0 006 639 B1

(12) **EUROPEAN PATENT SPECIFICATION**

(45) Date of publication of patent specification: 20.10.82

(51) Int. Cl.³: C 07 D 487/04 //(C07D487/04, 209/00, 205/00)

(21) Application number: 79102240.3

(22) Date of filing: 03.07.79

(54) **Crystalline N-formimidoyl thienamycin.**

(30) Priority: 03.07.78 US 921379

(43) Date of publication of application: 09.01.80 Bulletin 80/1

(45) Publication of the grant of the patent: 20.10.82 Bulletin 82/42

(84) Designated Contracting States: AT BE CH DE FR GB IT LU NL SE

(56) References cited:
DE - A - 2 652 679

The file contains technical information submitted after the application was filed and not included in this specification

(73) Proprietor: MERCK & CO. INC.
126, East Lincoln Avenue P.O. Box 2000
Rahway, New Jersey 07065 (US)

(72) Inventor: Miller, Thomas William
16 Vermont Avenue
Carteret, N.J. 07008 (US)

(74) Representative: Abitz, Walter, Dr.-Ing. et al,
Abitz, Morf, Gritschneder P.O. Box 86 01 09
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 006 639 B1

Crystalline N-formimidoyl thienamycin

Background of the Invention

This invention relates to crystalline N-formimidoyl thienamycin monohydrate and a process for its preparation.

The antibiotic N-formimidoyl thienamycin (I) is known, see for example, BE—A—848,545 (issued May 20, 1977):

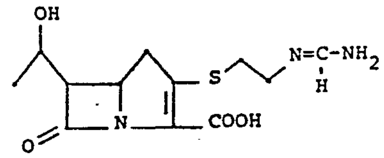

I

The crystalline form of N-formimidoyl thienamycin which is disclosed and claimed by this application is prepared from a lyophilized sample of I and is found to have unexpected stability in the solid state over the lyophilized form.

Detailed description of the Invention

The described and claimed crystalline N-formimidoyl thienamycin exists as a monohydrate and is unambiguously identified by the following parameters of the crystal which were obtained by X-ray powder diffraction.

| Peak Position | | Intensity | Peak Position | | Intensity |
|---|---|---|---|---|---|
| 2-Θ (CuKα) | d-Spacing (Å) | $I/I_o$ | 2-Θ (CuKα) | d-Spacing (Å) | $I/I_o$ |
| 9.75 | 9.07 | 100 | 26.9 | 3.31 | 75 |
| 11.35 | 7.80 | 40 | 28.7 | 3.11 | 37 |
| 13.85 | 6.39 | 11 | 29.9 | 2.99 | 17 |
| 14.5 | 6.11 | 4 | 30.8 | 2.90 | 7 |
| 15.75 | 5.63 | 14 | 31.85 | 2.81 | 12 |
| 17.5 | 5.06 | 30 | 32.6 | 2.75 | 10 |
| 18.9 | 4.69 | 27 | 32.9 | 2.72 | 10 |
| 19.6 | 4.53 | 12 | 33.4 | 2.68 | 5 |
| 20.0 | 4.44 | 14 | 33.9 | 2.64 | 10 |
| 21.45 | 4.14 | 30 | 34.8 | 2.58 | 26 |
| 21.75 | 4.08 | 36 | 35.6 | 2.52 | 14 |
| 22.3 | 3.98 | 45 | 37.0 | 2.43 | 5 |
| 22.9 | 3.88 | 30 | 38.3 | 2.35 | 9 |
| 23.3 | 3.82 | 28 | 39.3 | 2.29 | 6 |
| 24.3 | 3.66 | 33 | 40.0 | 2.25 | 9 |
| 25.35 | 3.52 | 24 | 42.0 | 2.20 | 14 |
| 25.8 | 3.45 | 20 | 42.4 | 2.14 | 18 |

Crystalline N-formimidoyl thienamycin monohydrate is prepared from a water/ethanol solution of N-formimidoyl thienamycin. The following specific example illustrates the crystallization.

Example 1

Crystalline N-formimidoyl thienamycin

A lyophilized sample of N-formimidoyl thienamycin (62 mg) is dissolved in 1.0 ml of water and diluted with 5.5 ml of 95% ethanol. The resulting solution is immersed in an ice-bath, stirred with a magnetic stirrer and seeded with N-formimidoyl thienamycin monohydrate crystals obtained by a procedure described immediately below. After $1\frac{1}{2}$ hours stirring, the crystals are recovered by centrifugation. After decantation, the crystals are washed with 1 ml of ethanol and dried under vacuum at 50°C for 1 hour to yield 56 mg of crystalline N-formimidoyl thienamycin monohydrate. The seed crystals employed in the above crystallization are prepared by the following procedure: A lyophilized sample of N-formidoyl thienamycin (24.5 mg) is dissolved in 0.5 ml of water, diluted to 3.0 ml with ethyl alcohol and stored in the freezer (—5°C). After two weeks, crystals are observed on the walls of the glass tube.

N-formimidoyl thienamycin is used as an antibiotic, as disclosed in the Belgian Patent 848,545, referred to above.

In the treatment of bacterial infections in man, the compound of this invention is administered orally or parenterally, in accordance with conventional procedures for antibiotic administration, in an amount of from about 2 to 600 mg/kg/day and preferably about 15 to 150 mg/kg/day in preferably divided dosage, e.g. three to four times a day. It may be administered in dosage units containing, for example 25, 250, 500 or 1000 mg. of active ingredient with suitable physiologically acceptable carriers or excipients. The dosage units are in the form of liquid preparations such as solutions or suspensions or as solids in tablets or capsules. It will, of course, be understood that the optimum dose in any given instance will depend upon the type and severity of infection to be treated, and the smaller doses will be employed for pediatric use, all of such adjustments being within the skill of the practioner in the field.

In addition to use alone, a particularly preferred method of using N-formimidoyl thienamycin is in combination with a dipeptidase (E.C. 3.4.13.11) inhibitor.

The class of dipeptidase inhibitor compounds can generally be described by the following formula:

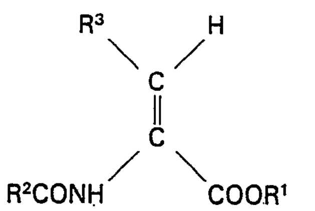

wherein $R^2$ and $R^3$ are hydrocarbon radicals in the range respectively of 3—10 and 1—15 carbon atoms. In either of these hydrocarbon radicals, a hydrogen may be replaced by halogen or a non-terminal methylene may be replaced by oxygen or sulfur, including oxidized forms of the latter. A hydrogen in $R^3$ may also be replaced by a carboxy, alkoxycarbonyl, amido, amidino, cyano, mercapto, and phosphono or amino group. $R^2$ is preferably a branched or cycloalkyl radical ($C_{3-10}$), with a limitation that the carbon adjacent to the carbonyl cannot be tertiary. $R^1$ is hydrogen, loweralkyl ($C_{1-6}$) or dialkylaminoalkyl (e.g., $—CH_2CH_2N(C_2H_5)_2$, $—CH_2CH(CH_3)N(CH_3)_2$).

A particularly preferred sub-group of inhibitor compounds are those in which $R^2$ is 2,2-dimethylcyclopropyl, and $R^3$ is alkyl of 1—15 carbon atoms, even more preferably $R^3$ is alkyl of 1—5 carbon atoms. The most preferred species of inhibitor presently is that in which $R^3$ is $C_5$ alkyl, $R^2$ is 2,2-dimethylcyclopropyl, and $R^1$ is hydrogen or sodium. This compound is Z-2-(2,2-dimethylcyclopropane-carboxamido)-2-octenoic acid, or sodium salt.

The most preferred administration route is parenteral, and the most preferred dosage form of the sodium Z-2-(2,2-dimethylcyclopropanecarboxamido)-2-octenoate with crystalline N-formimidoyl thienamycin is 75 or 150 mg of the former and 150 mg of the latter, coadministered by IV sterile aqueous solution injection, 3 or 4 times daily per human.

**Claim for the Contracting States: BE CH DE FR GB IT LU NL SE**

Crystalline N-formimidoyl thienamycin monohydrate.

**Claim for the Contracting State: AT**

A process of preparing crystalline N-formimidoyl thienamycin monohydrate which comprises forming a solution of lyophilized N-formimidoyl thienamycin in water/ethanol, stirring in the presence of a seed crystal of N-formimidoyl thienamycin monohydrate, and recovering the crystalline product thereby prepared.

**Revendication pour les Etats contractants: BE CH DE FR GB IT LU NL SE**

N-formimidoyl-thiénamycine cristalline monohydratée.

**Revendication pour l'Etat contractant: AT**

Procédé de préparation de N-formimidoyl-thiénamycine cristalline monohydratée dans lequel on forme une solution de N-formimidoyl-thiénamycine lyophilisée dans l'eau/éthanol, on agite en présence d'un cristal d'ensemencement de N-formimidoyl-thiénamycine monohydratée, et on recueille le produit cristallin ainsi préparé.

**Patentanspruch für die Vertragsstaaten: BE CH DE FR GB IT LU NL SE**

Kristallines N-Formimidoyl-thienamycin-monohydrat.

**Patentanspruch für den Vertragsstaat: AT**

Ein Verfahren zur Herstellung kristallinen N-Formimidoyl-thienamycin-monohydrats, umfassend die Bildung einer Lösung von lyophilisiertem N-Formimidoyl-thienamycin in Wasser/Ethanol, das Rühren in Gegenwart eines Impfkristalles von N-Formimidoyl-thienamycin-monohydrat, und die Gewinnung des dabei hergestellten kristallinen Produktes.